# EUROPEAN PATENT APPLICATION

(11) **EP 4 302 822 A1**
(43) Date of publication of application: **10.01.2024**
(21) Application number: 22207966.7
(22) Date of filing: 17.11.2022
(51) Int. Cl.: A61N 5/06

(54) **LOW-LEVEL LIGHT THERAPY DEVICE**

(30) Priority: 08.07.2022 WO PCT/CN2022/104636
(71) Applicant: Koninklijke Philips N.V., 5656 AG Eindhoven (NL)
(72) Inventor: GU, Wei, 5656AG Eindhoven (NL); GONG, Jian, 5656AG Eindhoven (NL); SU, Pu, 5656AG Eindhoven (NL); KONG, Tao, 5656AG Eindhoven (NL); DONG, Jia Ni, 5656AG Eindhoven (NL)
(74) Representative: Philips Intellectual Property & Standards

(57) **Abstract**

Proposed is a personal treatment device for performing low-level light therapy (LLLT) on a subject. Specifically, a connection assembly has been proposed that allows the releasable coupling of a light source module to a shell adapted to position the device to cover skin of the subject. The connection assembly allows the placement of the light source module in a plurality of different positions whilst ensuring an electrical connection of the light source module to an electrical power source. Thus, by the connection assembly, the light source module may be selectively placed to irradiate light toward a part of the subject's skin that requires treatment. In this way, fewer light sources may be required to achieve an improved efficacy of LLLT, without the need to provide additional light source modules.

## Description

### FIELD OF THE INVENTION

The present invention relates to the field of personal treatment devices, and in particular to the field of personal treatment devices that perform low-level light therapy.

### BACKGROUND OF THE INVENTION

Low-level light (laser) therapy (LLLT) is a form of treatment that applies low-level/energy lasers or light emitting diodes to a surface of a body. It has been shown that LLLT can relieve pain and stimulate cell function. For example, LLLT devices have been shown to be useful for treating musculoskeletal conditions, in dentistry, for wound healing, and to stimulate hair growth.

Typically, the efficacy of LLLT correlates with the number and density of light sources provided by the LLLT device. However, providing additional light sources also increases the cost of an LLLT device, as well as the power required to operate the LLLT device. Put another way, while providing more light sources may be beneficial for the efficacy of treatment, this solution also bring increased costs.

By way of specific example, LLLT devices used for hair loss treatment usually provide light sources across an inner surface of a shell/helmet for placement on a subject's head. LLLT devices for subjects with a higher (i.e. more severe) class of hair loss usually have a higher number or density of light source modules across the inner surface. However, as hair loss may be confined to specific areas of the subject's head, such a configuration often does not make full use of the provided light sources. In many cases, there may be too few light sources at hair loss locations, and too many light sources at non-hair-loss locations.

### SUMMARY OF THE INVENTION

The invention is defined by the claims.

According to examples in accordance with an aspect of the invention, there is provided a personal treatment device for performing low-level light therapy, LLLT, on a subject using an electrical power source, comprising:
a shell adapted to position the device to cover at least a portion of skin of a subject;
a light source module comprising at least one light source for LLLT, and configured to irradiate light toward the portion of skin; and
a connection assembly configured to releasably couple the light source module to the shell in a plurality of different positions, said coupling of the light source module to the shell effecting an electrical connection between the light source module and the electrical power source.

Proposed is a personal treatment device for performing low-level light therapy (LLLT) on a subject. Specifically, a connection assembly has been proposed that allows the releasable coupling of a light source module to a shell adapted to position the device to cover skin of the subject. The connection assembly allows the placement of the light source module in a plurality of different positions whilst ensuring an electrical connection of the light source module to an electrical power source. Thus, by the connection assembly, the light source module may be selectively placed to irradiate light toward a part of the subject's skin that requires treatment. In this way, fewer light sources may be required to achieve an improved efficacy of LLLT, without the need to provide additional light source modules.

Embodiments of the invention are based on the realisation that the effectiveness of LLLT is often dependent upon the density of light sources that irradiate light toward areas for treatment. Existing solutions simply provide additional light sources throughout a shell and/or helmet. Therefore, the capability to selectively couple a light source module at a plurality of locations on the shell may improve such devices, while avoiding costs associated with increasing the number of provided light sources at non target locations.

In other words, the invention provides a support surface (e.g. shell) that is adapted to cover a part of the skin of the subject (i.e. scalp, an arm, etc.). A connection assembly is provided that is capable of reversibly fixing a light source module to the support surface at each of a plurality of different positions, such that a user (i.e. the subject or a caregiver) may connect and disconnect the light source module to the shell at a desired/target position.

Furthermore, as the coupling of the light source module to the support surface effects an electrical connection between the light source module and an electrical power source, there may be no need for an individual power supply as part of the individual light source module (i.e. a battery supplied at the light source module). The electrical power source may thus be provided separately, and so may supply an appropriate power to the light source module. Yet further, this means that it may not be necessary to connect the light source module to the electrical power source manually (i.e. plugging a wire from the light source module to the electrical power source), improving simplicity of operation and the design of the personal treatment device.

In some embodiments, the releasable coupling of the light source module to the shell may be effected via a magnetic coupling between the light source module and the shell.

Magnetic coupling of the light source module to the shell provides a solution for coupling of the components that may be easy to operate. Further, as this solution may not require mechanical/moveable parts, the connection assembly may be more robust.

In some embodiments, the connection assembly may comprise a magnetic connector at the plurality of different positions, the magnetic connector comprising a magnetic material.

In such embodiments, the light source module may comprise an attachment magnet having an opposite pole to the magnetic material of the magnetic connector, or an attachment component comprising a ferromagnetic material. The attachment magnet and the attachment component may each be configured to couple the light source module to the magnetic connector at each of the plurality of different positions.

A magnet may thus be provided in the plurality of different positions, thus defining the plurality of different positions. In such a case, the light source module may have a counter part in order to achieve the magnetic coupling, whether this be by a magnet of a different pole, or a component comprising ferromagnetic material (i.e. a material that a magnet may releasably couple).

In alternative embodiments, the connection assembly may comprise a ferromagnetic connector at the plurality of different positions, the ferromagnetic connector comprising a ferromagnetic material. The light source module may comprise an attachment magnet comprising a magnetic material and may be configured to couple the light source module to the ferromagnetic connector at each of the plurality of positions.

As an alternative to the above, a ferromagnetic component/connector may be provided to define the plurality of positions. This may be a less expensive solution that a magnet defining the plurality of positions, as a magnet may only be provided on the light source module (i.e. only provided where a light source module is provided.

In some embodiments, the releasable coupling of the light source module to the shell may be effected by a complementary geometry between the light source module and the shell.

Instead of coupling the light source module to the shell using at least one magnet, a complementary geometry (i.e. a mechanical structure on the shell and light source module), which ensures a fit between the light source module and the shell. This may provide a cost effective means for releasable coupling the light source module to the shell.

In some embodiments, the plurality of different positions comprise a continuous plane of positions, and the connection assembly may be adapted to permit continuous movement of the light source module in the continuous plane of positions whilst maintaining the electrical connection between the the light source module and the electrical power source.

Indeed, it may improve ease of use of the device (i.e. ease of movement of the light source module relative to the shell) should the plurality of positions be continuous. This may allow a user to slide the light source module into a desired position, and may also allow more flexibility in the positioning of the light source module.

In some embodiments, the connection assembly may comprise a track defining the continuous plane of positions, and along which the light source module is movable while being releasably coupled to the track.

In other embodiments, the plurality of different positions may be discrete positions.

In this case, the plurality of different positions may be designed so as to target certain discrete areas. This may enable the light source module to couple in a sturdier fashion tan if the plurality of positions are continuous (i.e. by reducing the risk of accidental movement between positions). Further, this may reduce cost compared to the plurality of positons being continuous, as a coupling means only needs to be provided in discrete positions.

In some embodiments, the connection assembly may comprise a discrete connector component at each of the plurality of different discrete positions for releasably coupling the light source module to the shell.

In some embodiments, the connection assembly may further comprise at least one electrode for the electrical connection of the light source module to the electrical power source, and adapted to maintain contact with an electrical connector of the light source module when the light source module is in each of the plurality of different positions.

Thus, one solution for providing an electrical connection between the light source module to the electrical power source is an electrode pad that is provided such that it is in contact with an electrode of the light source module in each of the plurality of different positons. Such an electrode pad may be provided on a PCB for instance.

In some embodiments, the electrical connector of the light source module may comprise an anode connector and a cathode connector, and wherein the anode connector connects to a first electrode of the at least one electrode, and the cathode connector connects to a second electrode of the at least one electrode.

In some embodiments, the connection assembly may be provided on a printed circuit board (PCB). This may provide a cost effective and secure means for releasable coupling the light source module and the shell. Indeed, the shell may be a FPCB, or flexible PCB.

In some embodiments, the device may further comprise a housing for the electrical power source.

The electrical power source may be housed locally (i.e. as a battery pack) on the device, with the electrical power being transported to the light source module. This may improve a portability and ease of use of the device.

In some embodiments, the one or more light sources may be light emitting diodes or low-level lasers.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a side view of a personal treatment device for performing low-level light therapy (LLLT) according to a proposed embodiment;
Fig. 2 presents schematic diagrams of a connection assembly that utilizes a magnetic coupling of a light source module to a shell in a plurality of discrete positions according to an aspect of an exemplary embodiment;
Fig. 3 presents schematic diagrams of a connection assembly that utilizes a magnetic coupling of a light source module to a shell in a continuous plane of different positions according to an aspect of another exemplary embodiment;
Fig. 4 is a side view of a connection assembly for a personal treatment device that utilizes complementary geometries to effect a releasable coupling of a light source module to a shell according to an aspect of a further exemplary embodiment; and
Fig. 5 shows four different shells for positioning of a personal treatment device to cover skin of a subject, each having light source modules coupled in different positions.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

It should be understood that the Figures are merely schematic and are not drawn to scale. It should also be understood that the same reference numerals are used throughout the Figures to indicate the same or similar parts.

Proposed are personal treatment devices for low-level light/laser therapy (LLLT) of a subject. These devices irradiate low-level/energy light toward skin of the subject, as such light has been shown to stimulate cell function. Thus, LLLT devices have uses in many fields, such as hair regrowth, wound healing and pain relief.

Typically, LLLT devices are configured to cover an area of skin of the subject (i.e. a helmet over scalp) and irradiate light toward the area of skin covered by the device. Thus, the inside surface of these devices have a large number of light sources to provide an even wash of light for the skin of the user. Without knowing the treatment locations, the devices are provided with a larger number of light sources. However, this greatly increases the manufacturing cost of such devices, as well as a power required to operate such devices.

The proposed invention aims to overcome this issue by the provision of a connection assembly that is configured to releasably couple a light source module to a shell (covering skin of the subject) in a plurality of different positions, with such coupling effecting/causing an electrical connection between the light source module and an electrical power source. In this way, the light source module may be provided only where LLLT is required, reducing the number of light sources required for an effective treatment compared to an equal distribution of light sources across the shell. Indeed, the light source module may comprise many light sources in a dense arrangement, or many light source modules may be provided in a small area.

Thus, by utilizing a releasable coupling between the light source module and the shell, a user (i.e. a subject or a caregiver) may selectively place the light source module where treatment is required. This means that the light source module may be placed differently for changing needs of a subject, or for the different needs of different subjects. Additional/fewer light sources may also be provided as required by the user, due to the releasable nature of such a coupling.

Furthermore, as the releasable coupling effects/causes/creates an electrical connection between the light source module and the electrical power source, it may be easier to use the device. This is because no further steps may be required to provide power to the light source module other than placing the light source module in one of the plurality of different positions.

By way of explanation, in existing LLLT devices, anodes and cathodes of static light sources fixedly connect to an electrical power source by printed traces on a flexible printed circuit board (FPCB). However, in embodiments of the present invention, light source modules may be placed on a shell by a connection assembly that effects an electrical connection to a power source. The light source modules may therefore be placed freely in a plurality of different positions, such that the irradiated light may line up with a target position for treatment.

In some embodiments, the invention comprises the following components/features:
(i) a (conductive) connection assembly, having an anode pad and a cathode pad that may provide an electrical connection to a power source for the light source module when the light source module is placed in each of a plurality of different positions on a shell;
(ii) one or more light source modules, each module having an anode connector and a cathode connector suitable for connection to the anode pad and the cathode pad, respectively, when placed in each of the plurality of positions; and
(iii) a means by which the light source modules may be releasably coupled to the shell in each of the plurality of positions, such that the light source module may be moved to any of the plurality of positions.

Thus, embodiments of the invention provide a new hardware element for existing LLLT devices, by which light source modules may be releasably coupled to the shell in a plurality of different positions, effecting an electrical connection.

By way of specific example, the location of hair loss of subjects may differ greatly. The differing locations are attributable to different hair loss classes, while also varying subject-to-subject with the same hair loss class. Unfortunately, typical LLLT device designs do not reflect these differences, and simply provide an equal distribution of light sources that irradiate light equally to all parts of the scalp covered by the device.

In order to aid subjects with different hair loss classes and different hair loss conditions/locations, typical LLLT devices provide light units across the inner surface of the device, while increasing light source density for those with higher hair loss class levels. Such a configuration does not make full use of the light sources, with many light sources provided where they are not required (i.e. where hair loss is absent and/or minimal). Thus, often there are not enough light sources at hair loss locations, and there are too many light sources at non hair loss locations.

As an alternative example, subjects may have wounds at differing locations, and of differing sizes. While the device may be placed over the wound, much of the device may be placed over relatively healthy skin sites. Similarly to the above, many of the light sources of the device may irradiate light toward areas that do not require LLLT.

Accordingly, embodiments of the present invention enable movement of the light source modules, so that the light source module may be moved to irradiate site to an area of skin that requires treatment (i.e. a hair loss location, a wound location, etc.). Thus, embodiments both improve the efficacy of LLLT (i.e. a wound healing, a hair regeneration, etc.), while reducing a cost of the device.

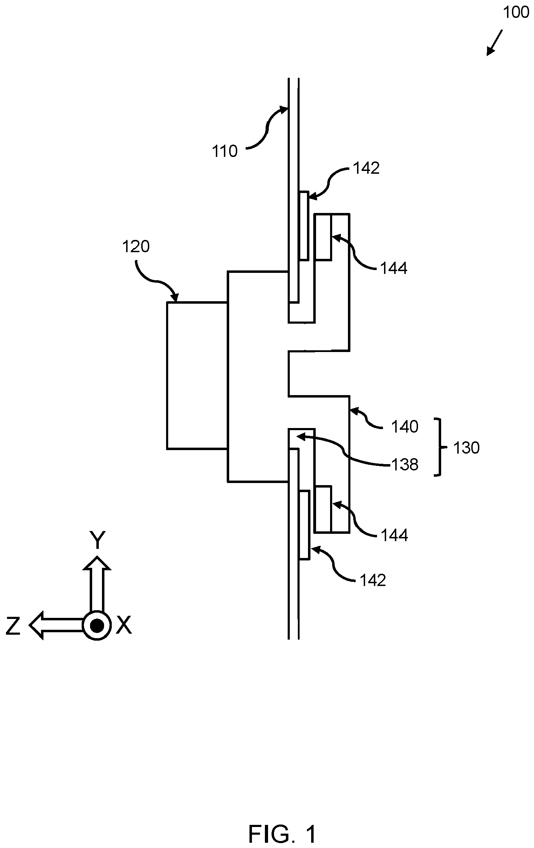

Referring now to Fig. 1, there is provided a schematic side view of a personal treatment device 100 for performing LLLT using an electrical power source, according to a proposed embodiment.

In some embodiments, the device may be a headset for performing LLLT on scalp of a subject. In this case, the shell 110 may take the form of a headset/helment suitable for placement on the subject's head. Alternatively, the device may be a fitted structure for performing LLLT on a portion of skin of the subject correspodning to a wound, or an area associated with pain. In this case, the shell 110 may take the form of a sleeve or other wearable that may be suitable for placement on a limb of the subject.

In any case, a shell 110 adapted to position the device to cover at least a portion of skin of a subject is provided. In other words, the shell 110 may be adapted to position/fit/fix such that the device is provided over/on the subject, such that a portion/area of skin is at least partially covered.

Fig. 1 contains a depiction of part of a shell 110 which corresponds to a single position of the plurality of different positions for coupling to the light source module 120. The shell 110 may take any form suitable for positioning to cover skin of the subject as explained above.

The shell 110 depicted in Fig. 1 is adapted to include part of a connection assembly 130. Indeed, the depicted shell 110 includes a gap to provide a complementary geometry with the light source module 120, as is explained in more detail below. However, it should be appreciated that in some embodiments the connection assembly 130 and the shell 110 may be separate components. For example, the shell 110 may be a plastic casing providing mechanical support for the connection assembly 130.

Moreover, a light source module 120 comprising at least one (i.e. one or more) light source for LLLT. There are a broad range of possible light sources suitable for performing LLLT, such as light emitting diodes (LEDs) and (low-level/energy) lasers. Such light sources emit lights at certain wavelength suitable for LLLT, which would be understood by the skilled person.

The light source module 120 is configured to irradiate light toward the portion of skin (i.e. in the Z direction, away from the shell). The light source module 120 may be configured to irradiate light toward only a part of the portion of skin covered by the shell 110 when the shell 110 is positioned over the portion of skin of the subject. In some embodiments, multiple light source modules may be provided in different locations of the plurality of different locations.

Further, a connection assembly 130 (i.e. an arrangement of components associated with the light source module 120 and the shell 110) configured to releasably/reversibly couple the light source module 120 to the shell 110 in a plurality of different positions (relative to the shell 110) is provided.

The coupling of the light source module 120 to the shell 110
effects/causes/creates/facilitates an electrical connection between the light source module 120 and an electrical power source. In other words, by placing the light source module 120 in each of the plurality of positons, the light source module 120 may be reversibly/releasably fixed in that position/location, while also being electrically connected to an electrical power source.

In Fig. 1, the light source module 120 is shown in one of the plurality of different positions. In the depicted embodiment, the connection assembly 130 couples the light source module 120 to the shell 110 via a tongue 140 and groove 138 configuration, in which a complementary geometry of the light source module 120 and the shell 110 provides a coupling between the light source module 120 and the shell 110. In other words, the tongue 140 is provided by a coupling component of the light source module 120, while the groove 138 is provided by a gap 138 in the shell 110.

For example, in the depicted embodiment, the light source module 120 may release from the shell 110 via twisting action (i.e. so that the tongue 140 of the light source module 120 moves from being substantially in the Y-direction to substantially in the X-direction, such that the tongue 140 may fit through the gap 138), or via sliding the tongue 140 of the light source module 120 to an open end of the gap 138 of the shell 110 (i.e. slid in the X-direction).

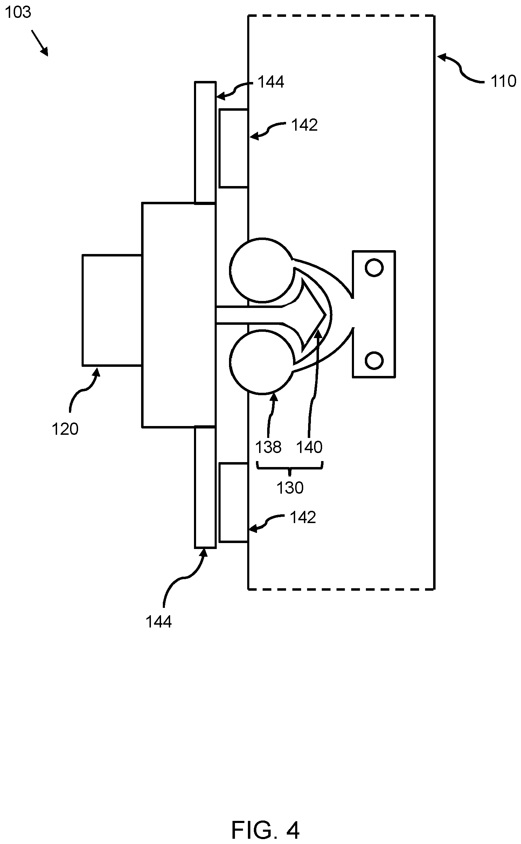

To reiterate, the depicted embodiment presents the releasable/reversible coupling/fixing of the light source module 120 to the shell 110 being effected/created by a complementary geometry between the light source module 120 and the shell 110. It should be understood that the skilled person would be aware of many different configurations that may achieve a reversible coupling by complementary geometries. Indeed, one such alternative configuration is described below in reference to Fig. 4.

Further, the depicted example also includes an anode 144 and a cathode 144 of the light source module 120. These may be connected (either physically or inductively) to electrode tracks 142. These electrode tracks 142 may facilitate the connection between the light source module 120 and the electrical power source when the light source module 120 is in each of the plurality of different positions.

The connection assembly 130 of Fig. 1 facilitates the coupling of the light source module 120 to the shell 110 at a plurality of different positions, wherein the plurality of different positions may be discrete or continuous. Indeed, the gap 138 in the shell 110 defining the groove 138 may be provided continually (for example, in the X direction). Thus, the gap 138 in the shell 110 may define a track along which the light source module 120 may be moved.

Alternatively, the gap 138 may be a small slot suitable for fitting a single light source module 120 at a single location. However, in this case, a plurality of gaps may be provided in the shell 110 at a plurality of different discrete positions.

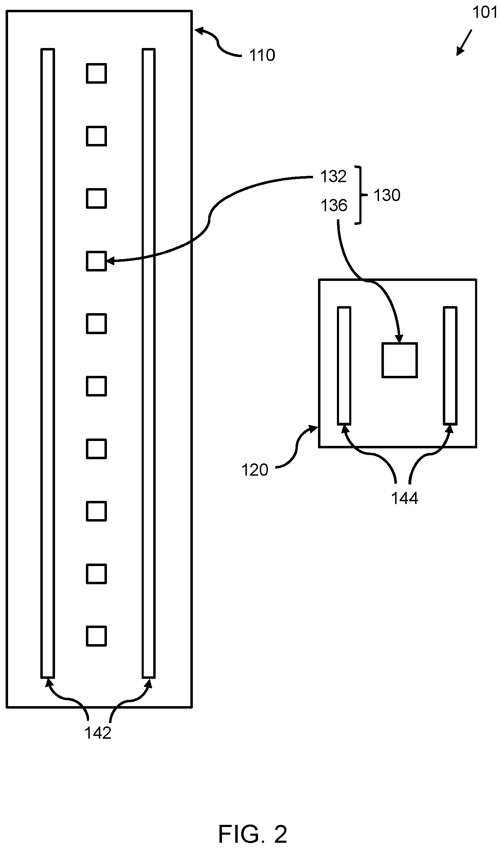

Moving on to Fig. 2, there is depicted a top view schematic of a connection assembly 130 that utilizes a magnetic coupling of a light source module 120 to a shell 110 in a plurality of different discrete positions according to an aspect of an exemplary embodiment. In this case, the releasable/reversible coupling of the light source module 120 to the shell 110 may be effected via a magnetic coupling between the light source module 120 and the shell 110.

Indeed, in order to achieve this the connection assembly 130 may comprise a magnetic connector 132 at the plurality of different positions, the magnetic connector 132 comprising a magnetic material.

In this case, the light source module 120 comprises either an attachment magnet 136 having an opposite pole to the magnetic material of the magnetic connector, or an attachment component 136 comprising a ferromagnetic material. Indeed, whether the attachment magnet 136 or the attachment component 136 are provided, both are configured to couple the light source module 120 to the magnetic connector 132 at each of the plurality of different positions.

Put another way, the light source module 120 may have the complementary component to the magnetic connector at the plurality of different positions, enabling a suitable coupling/fixing of the light source module 120 to the shell 110.

Alternatively, the connection assembly 130 may comprise a ferromagnetic connector 132 at the plurality of different positions, the ferromagnetic connector 132 comprising a ferromagnetic material. In other words, there is a component 132 provided at each of the plurality of positions that comprises a ferromagnetic material, being a material that is attracted to magnets.

In this case, the light source module 120 comprises an attachment magnet 136 comprising a magnetic material and configured to couple the light source module 120 to the ferromagnetic connector 132 at each of the plurality of positions.

To paraphrase the above, the releasable coupling of the light source module 120 to the shell 110 may be achieved via magnetic attraction. One of either the light source module 120 or the shell 110 may have a magnet. The shell 110 may have attached components 132 comprising either a magnetic or a ferromagnetic material at each of the plurality of positions in order to define the plurality of positions. The light source module 120 must then have the matching component 136 (i.e. a component comprising a magnet or ferromagnetic material) that complements the material used in the component 132 of the shell 110, thus enabling a coupling at the plurality of locations.

As shown in Fig. 2, the plurality of different positions may be discrete positions. In other words, the plurality of positions may be a number of different points, at which the light source module 120 may be coupled to the shell 110.

Thus, as shown, the connection assembly 130 may comprise a discrete connector component 132 at each of the plurality of different discrete positions for releasably coupling the light source module 120 to the shell 110. In the presently described embodiment, this is a different magnetic or ferromagnetic component 132 at each of the plurality of different positions. However, the discrete connector 132 may also be a connector 132 having a complementary geometry with a mechanical component 136 of the light source module 120 (thus enabling a coupling).

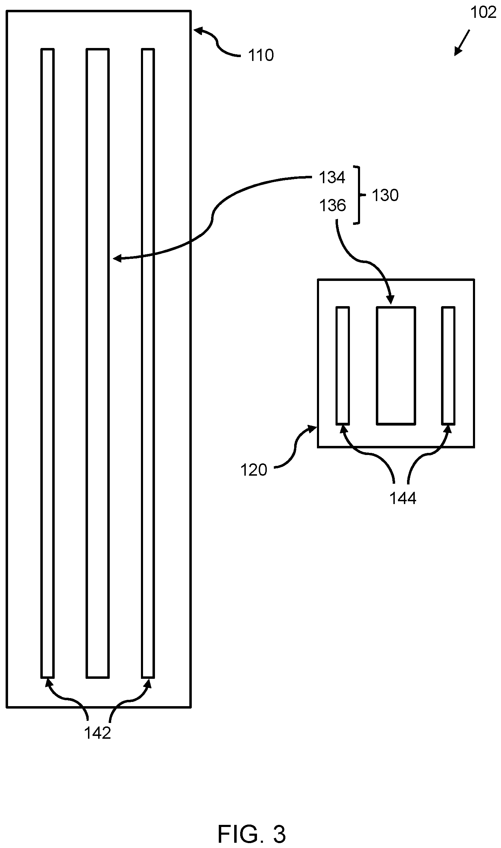

In reference now to Fig. 3, there is depicted a connection assembly 130 that utilizes a magnetic coupling of a light source module 120 to a shell 110 in a continuous plane of different positions according to an aspect of an exemplary embodiment.

The function of the various components, including the method of coupling the light source module 120 to the shell 110 is similar to Fig. 2. However, the connection assembly 130 comprises one continuous track 134 of magnetic or ferromagnetic material defining the plurality of positons.

This demonstrates that, in some embodiments, the plurality of different positions comprise a continuous plane of positions. In this case, the connection assembly 130 is adapted to permit continuous movement of the light source module 120 in the continuous plane of positions whilst maintaining the electrical connection between the the light source module 120 and the electrical power source. In other words, the light source module 120 may be able to slide between the plurality of different positions while still being connected to the electrical power source.

Indeed, the connection assembly 130 may comprises a track 134 defining the continuous plane of positions, and along which the light source module 120 is movable while being releasably coupled to the track. This is the case in Fig. 3, where a single track 134 is depicted. However, it may also be appreciated that multiple tracks may be provided.

Moreover, in reference to both Figs. 2 and 3, there are provided two electrode tracks 142 on the shell 110. These electrode tracks 142 may be connected on at least one end to a power source (not shown). In this way, the electrode tracks 142 may be suitable for facilitating the electrical connection of the light source module 120 to the electrical power source. The electrode tracks 142 may maintain contact with electrical connectors 144 of the light source module 120 when the light source module 120 is in each of the plurality of different positions.

Further, in the presented embodiment, the electrical connectors 144 of the light source module 120 comprise an anode connector and a cathode connector. The anode connector connects to a first electrode of the at least one electrode, and the cathode connector connects to a second electrode of the at least one electrode. The connection may be physical, or may be inductive. In any case, the electrical connection may be facilitated, such that the light source module 120 is provided with power to operate the one or more light sources.

Fig. 4 presents an a side view of a connection assembly 130 for a personal treatment device that utilizes complementary geometries to effect a releasable coupling of a light source module 120 to a shell 110 according to an aspect of a further exemplary embodiment.

In the depicted embodiment, there is provided a light source module 120 configured to irradiate light toward the portion of skin by one or more light sources suitable for LLLT. The light source module 120 has an anode 144 and a cathode 144 suitable for connection to electrode tracks 142 (i.e. provided on the shell) for connection to an electrical power source (not shown). The anode 144 and cathode 144 may connect to the electrode tracks 142 using any method known in the art.

Also, there is provided a shell 110 adapted to position the device to cover at least a portion of skin of a subject. The shell 110 may be of any shape suitable for positioning on the subject, and may optionally house the electrical power source. On the shell 110 there is provided a component of a connection assembly 130 configured to releasably couple the light source module 120 to the shell 110 in one of the plurality of different positions. Further connection assembly 130 components are not presented here for the sake of simplicity. However, it should be appreciated that may connection assembly 130 components may be provided on the shell 110, each defining a different position.

The depicted connection assembly 130 is based on a releasable mechanical coupling assembly. The arms of the component 138 fixedly connected to the shell 110 may be flexible, such that a complementary geometry 140 connected to the light source module 120 may click into position between the ends of the arms, be held in potion, and be released when a suitable force is applied.

Indeed, it should be understood that this is one mechanical solution for defining one of the plurality of different positions. As described above, alternative options include complementary magnets, or a tongue and groove configuration. Further modifications and alternative embodiments for the connection assembly 130 would be appreciated by the skilled person, in so far as they may define a releasable coupling of the light source module 120 to the shell 110, while also effecting an electrical connection between the light source module 120 and an electrical power source.

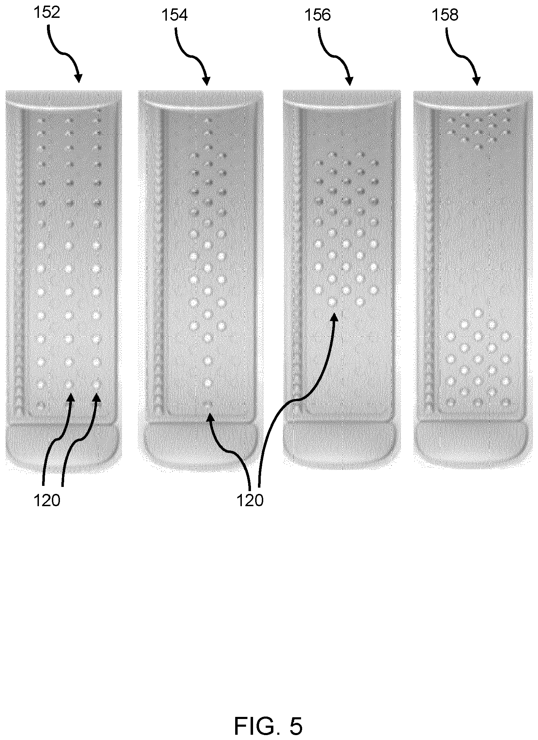

Fig. 5 presents multiple shells for positioning of a personal treatment device to cover skin of a subject, each having light source modules 120 coupled in different positions. As presented, there are a plurality of different locations on the shell for coupling the light source module 120.

In the depicted examples, there are also a plurality of light source modules 120 that may be placed in different positions in the plurality of different positions. The first shell 152 presents a configuration where the light sources modules are distributed evenly throughout the shell. The second shell 154, third shell 156 and fourth shell 158 depict different potential configurations of the placements of the light source modules 120 that may be more suitable when an even distribution is not desirable (i.e. targeting a hair loss site, or a wound).

Thus, provided are devices that enable appropriate distribution of the light source modules 120 by a connection assembly 130 that allows releasable/reversible coupling of the light source module 120 to the shell at a plurality of different positons. In this way, an efficacy of LLLT treatment by the device may be improved while reducing costs associated with providing a dense, even, distribution of the light sources across a whole surface of a shell.

## Claims

1. A personal treatment device for performing low-level light therapy, LLLT, on a subject using an electrical power source, comprising:
a shell (110) adapted to position the device to cover at least a portion of skin of a subject;
a light source module (120) comprising at least one light source for LLLT, and configured to irradiate light toward the portion of skin; and
a connection assembly (130) configured to releasably couple the light source module to the shell in a plurality of different positions, said coupling of the light source module to the shell effecting an electrical connection between the light source module and the electrical power source.

2. The device of claim 1, wherein the releasable coupling of the light source module (120) to the shell (110) is effected via a magnetic coupling between the light source module and the shell.

3. The device of claim 2, wherein the connection assembly (130) comprises a magnetic connector (132, 134) at the plurality of different positions, the magnetic connector comprising a magnetic material.

4. The device of claim 3, wherein the light source module (120) comprises an attachment magnet (136) having an opposite pole to the magnetic material of the magnetic connector, or an attachment component (136) comprising a ferromagnetic material, wherein either one the attachment magnet and the attachment component are each configured to couple the light source module to the magnetic connector at each of the plurality of different positions.

5. The device of claim 2, wherein the connection assembly (130) comprises a ferromagnetic connector (132, 134) at the plurality of different positions, the ferromagnetic connector comprising a ferromagnetic material, and the light source module (120) comprises an attachment magnet (136) comprising a magnetic material and configured to couple the light source module to the ferromagnetic connector at each of the plurality of positions.

6. The device of claim 1, wherein the releasable coupling of the light source module to the shell is effected by a complementary geometry (138, 140) between the light source module and the shell.

7. The device of any of claims 1-6, wherein the plurality of different positions comprise a continuous plane of positions, and wherein the connection assembly (130) is adapted to permit continuous movement of the light source module (120) in the continuous plane of positions whilst maintaining the electrical connection between the the light source module and the electrical power source.

8. The device of claim 7, wherein the connection assembly (130) comprises a track (134) defining the continuous plane of positions, and along which the light source module (120) is movable while being releasably coupled to the track.

9. The device of any of claims 1-6, wherein the plurality of different positions are discrete positions.

10. The device of claim 9, wherein the connection assembly (130) comprises a discrete connector component (132) at each of the plurality of different discrete positions for releasably coupling the light source module (120) to the shell (110).

11. The device of any of claims 1-10, wherein the connection assembly (130) further comprises at least one electrode (142) for the electrical connection of the light source module (120) to the electrical power source, and adapted to maintain contact with an electrical connector (144) of the light source module when the light source module is in each of the plurality of different positions.

12. The device of claim 11, wherein the electrical connector (144) of the light source module comprises an anode connector and a cathode connector, and wherein the anode connector connects to a first electrode of the at least one electrode (142), and the cathode connector connects to a second electrode of the at least one electrode.

13. The device of any of claims 1-12, wherein the connection assembly (130) is provided on a printed circuit board.

14. The device of any of claims 1-13, further comprising a housing for the electrical power source.

15. The device of any of claims 1-14, wherein the one or more light sources are light emitting diodes or low-level lasers.
